# FASCICULE DE BREVET EUROPEEN

(11) **EP 1 883 452 B1**
(45) Date de publication et mention de la délivrance du brevet: **19.12.2012**
(21) Numéro de dépôt: 06755932.8
(22) Date de dépôt: 15.05.2006
(51) Int. Cl.: A61P 31/12, A61P 33/00, A61P 33/10, A61P 35/00, A61K 45/06, A61K 31/045, A61K 31/05

(54) **COMPOSITION PHARMACEUTIQUE COMPRENANT UN AGENT ANTIPARASITAIRE ET UN ACTIF CHOISI PARMI LE THYMOL, L'EUGÉNOL, ET LE CARVACROL**
PHARMAZEUTISCHE ZUSAMMENSETZUNG ENTHALTEND EIN ANTIPARASITIKUM UND EINE AKTIVE VERBINDUNG AUSGEWÄHLT AUS THYMOL, EUGENOL ODER CARVACROL
PHARMACEUTICAL COMPOSITION CONTAINING AN ANTI PARASITIC AGENT AND AN ACTIVE SELECTED FROM THYMOL, EUGENOL OR CARVACROL

(30) Priorité: 13.05.2005 WO PCT/IB2005/001314
(43) Date de publication de la demande: 06.02.2008
(73) Titulaire: Advanced Scientific Developments, 20200 Casablanca (MA)
(72) Inventeur: REMMAL, Adnane, Adarissa Fes, 30000 (MA)
(74) Mandataire: Gallois, Valérie
(86) Numéro de dépôt international: PCT/IB2006/001441
(87) Numéro de publication internationale: WO 2006/120568

(56) Documents cités:
- WO-A-01/58486
- WO-A-96/40192
- WO-A-97/35839
- WO-A-03/069993
- US-A1- 2003 064 948
- US-A1- 2003 225 003
- BAILENGER, J. ET AL: "A new anthelmintic : piperazine thymolsulfonate . I. Experimental study" BULLETIN DES TRAVAUX DE LA SOCIETE DE PHARMACIE DE BORDEAUX , 93, 161-6 CODEN: BTSPA9; ISSN: 0366-3825, 1955, XP008079770
- OELKERS, H. A.: "The mechanism of the anthelmintic action of piperazine" ARZNEIMITTEL-FORSCH. , 7, 329-30, 1957, XP008079795
- Annexe: Résultats complémentaires

## Description

L'invention concerne une composition pharmaceutique comprenant deux substances thérapeutiquement actives dont l'une exerce une action de potentialisation sur l'autre, ainsi que l'utilisation de cette composition.

Il est connu que l'efficacité des agents thérapeutiques dépend des doses utilisées, ce qui oblige, dans le cas des résistances partielles à augmenter les doses des agents thérapeutiques pour atteindre l'efficacité recherchée. Cette augmentation de la dose conduit à des problèmes d'apparition d'effets secondaires indésirables et de toxicité aiguë ou chronique, pouvant compliquer considérablement l'état des patients traités.

Cette résistance partielle peut devenir une résistance totale. Dans ce cas, l'augmentation des doses n'a plus aucun effet thérapeutique bénéfique, seuls les effets de toxicité sont observés. Le traitement consiste alors à changer l'agent thérapeutique.

Cette cascade peut se répéter et conduire à la situation la plus grave: la résistance totale à de multiples agents thérapeutiques (multi-drug resistance).

Ainsi, en particulier, les malades immunodéprimés, deviennent de plus en plus difficiles à traiter et leur espérance de vie en est réduite d'autant. De plus, leur confort de vie est largement affecté par l'administration à hautes doses d'agents thérapeutiques.

L'invention a pour but de pallier ces problèmes en proposant d'associer au moins deux substances thérapeutiquement actives, dont l'une potentialise l'activité de l'autre, ce qui permet non seulement d'abaisser les doses de chaque substance thérapeutiquement active mais également de traiter les patients atteints d'infections à germes résistants. A cet effet, l'invention propose une composition pharmaceutique caractérisée en ce qu'elle comprend:
- au moins une première substance thérapeutiquement active choisie parmi le thymol, l'eugénol le carvacrol et les isomères et mélanges de ceux-ci, et
- au moins une seconde substance thérapeutiquement active qui est un antiparasitaire.

La première substance thérapeutique peut être obtenue par synthèse chimique ou à partir d'une source végétale.
L'antiparasitaire entrant dans la composition de l'invention est choisi parmi les protozoocides, et leurs mélanges. Des antiparasitaires connus sont la salynomicine, le niclosamide, le praziquantel ou isoquinoléine, l'albendazole, le fubendazole, le mébendazole, le tiabendazo, le triclabendazole, le bithionol, le diéthylcarbamazine, l'ivermectine, la lévamisole, le métrifonate, le niclofan, l'oxamniquine, une pipérazine, le pyrantel, le pyrvinium, le métronidazole, le nimorazole, l'ornidazole, le secnidazole, le tinidazole, le méglumine antimoniate, le pentamidine iséthionate, la stibogluconate de sodium, le benznidazole, la difluorométhiylornithine (DFMO), le mélarsoprol, le nifurtimox, la suramine sodique, l'amodiaquine, l'artémisine, l'artésunate et leurs dérivés, la chloroquine, la doxycycline, l'halofantrine, la méfloquine, la primaquine, le proguanil, la pyronaridine, la quinine, l'atovaquone, l'azithromycine, la clarithromycine, la clindamycine, le cotrimoxazole, le dapsone, la déhydroémétine, la paromomycine, la pyriméthamine, la spiramicine, la sulfadiazine, le ténonitrozole, le tillquinol triméthoprime, le triméthrexate, et leurs dérivés et mélanges.

Une composition antiparasitaire particulièrement préférée est une composition dans laquelle la première substance thérapeutiquement active est le thymol et l'antiparasitaire est la salynomicine.
Une autre composition antiparasitaire particulièrement préférée est une composition antiparasitaire dans laquelle ladite première substance thérapeutiquement active est le carvacrol et l'antiparasitaire est la salynomicine.

Encore une autre composition particulièrement préférée est une composition antipaludéenne dans laquelle ladite première substance thérapeutiquement active est choisie parmi le carvacrol et l'eugénol, et l'antiparasitaire est un antipaludéen choisi parmi la méfloquine, la chloroquine, et l'artésunate, et les mélanges de ceux-ci.

L'invention propose également une trousse (kit) caractérisée en ce qu'elle contient au moins un premier récipient contenant une première substance thérapeutiquement active choisie parmi le thymol, l'eugénol, le carvacrol et les isomères et mélanges de ceux-ci, et au moins un second récipient contenant une seconde substance thérapeutiquement active qui est un antiparasitaire.

L'invention propose encore un traitement d'une affection due à un parasite caractérisée en ce qu'on administre à un patient atteint d'une affection due à un parasite, de manière simultanée ou séquentielle au moins une première substance thérapeutiquement active choisie parmi le thymol, l'eugénol le carvacrol, et les isomères et mélanges de ceux-ci, et au moins une seconde substance thérapeutiquement active qui est un agent antiparasitaire.

Dans cette méthode, de préférence, on administre de manière simultanée ou séquentielle, à un patient atteint d'une affection due â un parasite entre 10 et 100 mg/kg de poids du patient/jour de ladite première substance thérapeutiquement active, et entre 20 et 100 mg/kg de poids du patient/jour de ladite seconde substance thérapeutiquement active qui est un antiparasitaire.

De préférence, dans cette méthode, ladite première substance thérapeutiquement active est choisie parmi le carvacrol, l'eugénol et le thymol et ladite seconde substance thérapeutiquement active est la salynomycine.

L'invention propose également un traitement du paludisme caractérisée en ce qu'on administre à un patient atteint de paludisme, de manière simultanée ou séquentielle, au moins une première substance thérapeutiquement active choisie parmi l'eugénol ou le carvacrol, et au moins une seconde substance thérapeutiquement active qui est un antipaludéen choisi parmi la méfloquine, l'artésunate, la chloroquine et leurs mélanges.

L'invention sera mieux comprise et d'autres buts et avantages de celle-ci apparaîtront plus clairement à la lecture de la description explicative qui suit.

La composition pharmaceutique de l'invention comprend en tant que première substance thérapeutiquement active le thymol, l'eugénol, le carvacrol, et leurs isomères ainsi que leurs mélanges.

Ces substances doivent être pures.

Ces composés ont des propriétés antimicrobiennes bien connues en eux-mêmes.

Le thymol, l'eugénol, le carvacrol se trouvent en proportions variés dans différents extraits de plantes aromatiques, c'est-à-dire qu'ils peuvent être purifiés à partir de ces plantes. Cependant, ils peuvent être tout simplement obtenus par synthèse chimique.

Or, les inventeurs ont maintenant découvert que ces composés ont un effet de potentialisation sur de nombreuses substances thérapeutiquement actives dont les agents antiparasitaires connus et déjà utilisés en tant que médicament spécifique de cette spécialité.

La seconde substance thérapeutiquement active comprise dans la composition pharmaceutique de l'invention est donc un antiparasitaire, qui est déjà connu en tant que tel et déjà utilisé en tant que médicament spécifique de cette spécialité, et dont l'activité est potentialisée.

Les antiparasitaires connus et déjà utilisés en tant que médicament spécifique de cette spécialité qui sont utilisés dans la composition pharmaceutique de l'invention, et dont l'effet sera potentialisé par la première substance pure thérapeutiquement active, appartiennent à famille des protozoocides.

La famille des anthelminthiques comprend les cestocides tels que le niclosamide et le praziquantel, les benzimidazoles tels que l'albendazole, la flubendazole, le mébendazole, le tiabendazole, le triclabendazole, et d'autres anthelminthiques, tels que le bithionol, le diéthylcarbamazine, l'ivermectine, la lévamisole, le métrifonate, le niclofan, l'oxamniquine, la pipérazine, le pyrantel, le pyrvinium.

La famille des protozoocides comprend la série des 5-nitro-imidazoles, le métronidazole, le nimorazole, l'ornidazole, le secnidazole, le tinidazole, les leishmanocides, tels que le méglumine antimoniate, le pentamidine iséthionate, la stibogluconate de sodium, les trypanosomicides, tels que le benznidazole, la difluorométhylornithine (DFMO), le mélarsoprol, le nifurtimox, la suramine sodique, les antipaludéens, tels que l'amodiaquine, l'artémisine, la chloroquine, la doxycycline, l'halofantrine, la méfloquine, la primaquine, le proguanil, la pyronaridine, la quinine, l'artésunate. D'autres protozoocides sont la salynomicine, l'atovaquone, l'azithromycine, la clarithromycine, la clindamycine, le cotrimoxazole, le dapsone, la déhydroémetine, la paromomycine, la pyriméthamine, la spiramicine, la sulfadiazine, le ténonitrozole, le tillquinol triméthoprime,le triméthrexate.

Ces composés peuvent être utilisés seuls, ou en combinaison l'un avec l'autre.

L'invention telle que revendiquée s'adresse à la salynomicine, la méfloquine, la chloroquine et l'artésunate utilisées en combinaison avec le carvacrol, l'eugénol et/ou le thymol.

En dehors de la présente invention, étant donné l'effet potentialisateur exercé par la première substance thérapeutiquement active définie dans l'invention, d'autres agents antiparasitaires connus ou à venir pourraient également être utilisés avec succès.

La composition pharmaceutique selon l'invention peut être formulée sous une forme adaptée pour une administration simultanée ou séquentielle desdites au moins première et seconde substances thérapeutiquement actives.

La forme galénique de la composition pharmaceutique de l'invention sera adaptée à son utilisation.

Par exemple, elle pourra être utilisée sous la forme de solution, de suspension, de cachet ou autres.

Les compositions pour administration parentérale sont généralement des solutions ou des suspensions stériles pharmaceutiquement acceptables qui peuvent éventuellement être préparées extemporanément au moment de l'emploi.

Pour la préparation de solutions ou de suspensions non aqueuses, on peut utiliser des huiles végétales naturelles telles que l'huile d'olive, l'huile de sésame ou l'huile de paraffine ou les esters organiques injectables tels que l'oléate d'éthyle. Les solutions stériles aqueuses peuvent être constituées d'une solution des substances thérapeutiquement actives dans l'eau. Les solutions aqueuses conviennent pour l'administration intraveineuse dans la mesure où le pH est convenablement ajusté et où l'isotonicité est réalisée, par exemple par ajout d'une quantité suffisante de chlorure de sodium ou de glucose.

En effet, étant donné la structure chimique des antiparasitaires, et d'autre part, vu la structure chimique du carvacrol, du thymol, de l'eugénol, on pense, mais sans vouloir être lié par cette théorie, que le carvacrol, le thymol, l'eugénol et leurs isomères et mélanges, interagissent avec les antiparasitaires, pour former des complexes ayant une structure qui diffuse plus facilement dans les liquides physiologiques de l'organisme et qui diffuse plus facilement dans le cytoplasme des cellules infectées ciblées.

Mais, il a été démontré que lorsque les différents éléments de la composition pharmaceutique de l'invention sont mélangés en présence de détergents tels que le Tween ou le Triton ou de dissolvants tels que l'éthanol et le DMSO (diméthyl sulphoxide), les molécules actives de la première et de la seconde substance thérapeutiquement active s'associent avec les molécules de détergents et de dissolvants et ne forment pas de complexe de potentialisation.

Or on a découvert que le complexe de potentialisation se forme lorsqu'on utilise une suspension aqueuse d'agar, en tant que moyen de dispersion par viscosité.

Ainsi, la composition pharmaceutique de la présente invention sera de préférence préparée sans détergent et sans solvant. Par exemple, elle sera mise en suspension aqueuse rendue visqueuse par de l'agar à une concentration non gélifiante, par exemple de 1 à 5 grammes d'agar par litre de suspension.

La composition pharmaceutique de l'invention permet de traiter des infections localisées ou systémiques à germes résistants avec des doses plus faibles de chacune desdites première et seconde substances thérapeutiquement actives que les doses nécessaires au traitement des mêmes infections à germes sensibles, par l'une ou l'autre de ces mêmes dites première et seconde substances thérapeutiquement actives seules.

En effet la composition de l'invention permet d'utiliser des doses de ladite première substance thérapeutiquement active, lorsque en combinaison avec ladite seconde substance thérapeutiquement active, environ trois fois inférieures à celles nécessaires lorsque ladite première substance thérapeutiquement active est utilisée seule et des doses de ladite seconde substance thérapeutiquement active, lorsque en combinaison avec ladite première substance thérapeutiquement active, de 2 à 10 fois inférieures à celles nécessaires lorsque ladite seconde substance thérapeutiquement active est utilisée seule.

Ceci a pour conséquence d'offrir un traitement qui présente les avantages suivants:
- efficacité contre les germes sensibles avec des doses très faibles,
- efficacité contre les germes résistants à un agent thérapeutique,
- efficacité contre les germes résistants à plusieurs agents thérapeutiques,
- lutte contre les phénomènes de récidive,
- lutte contre les phénomènes de sélection de germes résistants,
- dans tous ces cas, diminution des risques de toxicité et/ou apparition d'effets secondaires indésirables, bien connus de l'homme du métier, par l'administration de doses très faibles.

De plus, il en résulte une diminution du coût de production du traitement étant donné la faible quantité de principes actifs utilisés.

Les compositions pharmaceutiques de l'invention peuvent se présenter sous la forme de liposomes ou sous forme d'association avec des supports tels que les cyclodextrines ou les polyéthyléneglycols.

Les compositions pharmaceutiques de l'invention représentent un moyen simple et efficace pour lutter contre les problèmes liés aux agents microbiens en général qui sont essentiellement la résistance aux agents thérapeutiques et la toxicité de ceux-ci générée par l'utilisation de fortes doses.

En effet, le thymol, l'éugénol le carvacrol, et leurs mélanges et isomères, sont des molécules simples n'ayant jamais été reportées comme ayant une toxicité quelconque et leur ajout ayant un effet potentialisateur sur la seconde substance thérapeutiquement active permet d'utiliser des doses beaucoup plus faibles de cette seconde substance thérapeutiquement active.

Le procédé de traitement des patients atteints d'une affection parasitaire consistera donc, dans une première variante, à administrer à ces patients la dose déterminée par le médecin de la composition pharmaceutique de l'invention contenant les doses appropriées de ladite au moins une première substance thérapeutiquement active, combinées aux doses appropriées de ladite au moins une seconde substance thérapeutiquement active, c'est-à-dire l'antiparasitaire approprié.

Dans une seconde variante, le procédé de traitement des patients atteints d'une affection parasitaire consistera à administrer à ces patients séquentiellement la dose déterminée par le médecin de ladite au moins une première substance thérapeutiquement active, puis la dose appropriée de ladite au moins une seconde substance thérapeutiquement active, c'est-à-dire l'antiparasitaire approprié ou l'inverse.

A cet effet, l'invention propose une trousse contenant, au moins un premier récipient contenant une desdites premières substances thérapeutiquement actives, et au moins un second récipient contenant une desdites secondes substances thérapeutiquement actives.

Cette trousse permettra au personnel soignant de préparer à la demande soit un mélange, en les doses appropriées, de(s) la première(s) substance thérapeutique voulue(s) et de(s) l'antiparasitaire(s) voulu(s), pour une administration simultanée, soit d'administrer séquentiellement et de façon séparée la dose appropriée de ladite au moins une première substance thérapeutiquement active, puis la dose appropriée de ladite au moins une seconde substance thérapeutiquement active, c'est-à-dire l'antiparasitaire approprié, ou l'inverse.

Cependant, on préférera utiliser un mélange pour utilisation simultanée pour permettre au complexe de potentialisation de se former et d'agir Immédiatement dès l'administration au patient.

Pour mieux faire comprendre l'invention, on va maintenant décrire à titre d'exemples purement illustratifs et non limitatifs plusieurs modes de mise en oeuvre.

### EXEMPLE 1 : ● Traitement de la coccidiose,chez des poulets avec la salynomicine potentialisée par le carvacrol ou le thymol.

Ces tests sont des tests *in vivo* qui ont consisté à déterminer le gain de poids, l'indice de consommation, l'excrétion quotidienne d'oocystes et l'aspect des matières fécales chez les poulets traités.

L'expérience a été menée sur un échantillon de 200 poulets âgés de 22 jours (poids 620 g en moyenne) souffrant d'une coccidiose subclinique provenant d'une bande de 10 000 poulets de chair. La coccidiose subclinique a été diagnostiquée le 20^{ème} jour. L'excrétion d'oocystes chez les animaux de l'échantillon était en moyenne de l'ordre de 50 000 oocystes/g (OPG).

L'analyse microscopique a révélé l'existence de deux espèces *d'Eimeria: Eimeria acervulina* et *Emeria tenella.*

L'antiparasitaire testé est la salynomycine qui fait partie des agents antiparasitaires les plus utilisés pour ce genre d'affection. Deux compositions pharmaceutiques antiparasitaires selon l'invention ont été fabriquées en mélangeant la salynomycine à différentes concentrations, avec le carvacrol ou le thymol à une concentration infra-inhibitrice de 75 mg/kg d'aliment.

L'activité antiparasitaire a également été testée soit avec la salynomycine seule, soit avec du carvacrol seul soit avec du thymol seul. L'activité antiparasitaire a été mesurée par la détermination de critères classiquement retenus dans ce genre d'affection qui sont : le gain de poids, l'indice de consommation, l'excrétion quotidienne d'oocystes et l'aspect des matières fécales. Ces critères ont été notés au cinquième jour (juste après la fin du traitement) et au quinzième jour (dix jours après la fin du traitement).

L'échantillon a été subdivisé en 7 lots de 28 poulets chacun :
Lot 1 : Animaux non infectés et non traités (témoins sains) poids au 22^{ème} jour: 750 g en moyenne.
Lot 2: Animaux infectés non traités (témoin d'affection).
Lot 3: Animaux infectés traités à la salynomycine à une dose de 40 mg/kg d'aliment.
Lot 4: Animaux infectés traités au carvacrol à 75 mg/kg d'aliment.
Lot 5: Animaux infectés traités au thymol à 75 mg/kg d'aliment.
Lot 6: Animaux infectés traités à la salynomycine à une concentration de 40 mg/kg d'aliment potentialisée avec du carvacrol à une concentration de 75mg/kg d'aliment.
Lot 7: Animaux infectés traités à la salynomycine à une concentration de 40 mg/kg d'aliment potentialisée par le thymol à une concentration de 75mg/kg d'aliment.

L'aliment utilisé est un aliment industriel (finition) ne contenant pas d'agent antiparasitaire.

Les agents antiparasitaires utilisés ont été mélangés avec cet aliment pendant les cinq jours de traitement. Les animaux avaient un accès libre à l'aliment pendant toute la durée de l'expérience.

Le tableau 1 ci-après donne les résultats du test qui compare l'action des compositions selon l'invention par rapport à la salynomycine seule, au carvacrol seul et au thymol seul.

**Tableau 1**

| | Gain de poids (Grammes ± ESM) | | Indice de consommation | | Sécrétion d'oocystes (OPG ± ESM) | | Aspect des fientes | |
|---|---|---|---|---|---|---|---|---|
| | 5 jours | 15 jours | 5 jours | 15 jours | 5 jours | 15 jours | 5 jours | 15 jours |
| Lot 1 | 250 ± 24 | 1000 ± 70 | 1,62 | 1,82 | 1200 ± 158 | 1500 ± 115 | normal | normal |
| Témoins non infectés non traités | | | | | | | | |
| Lot 2 | 168 ± 42 | 780 ± 97 | 2,3 | 3,1 | 92000 ± 5500 | 194 000 ± 7800 | diarrhéique | diarrhéique |
| Témoins infectés non traités | | | | | | | | |
| Lot 3 | 182 ± 28 | 835 ± 44 | 2,2 | 2,9 | 110000 ± 6700 | 164000 ± 7200 | diarrhéique | diarrhéique |
| Traitement à la salynomicine (40 mg/kg d'aliment) | | | | | | | | |
| Lot 4 | 176 ± 32 | 812 ± 51 | 2,4 | 3 | 105000 ± 5900 | 147000 ± 4300 | diarrhéique | diarrhéique |
| Traitement au carvacrol seul (75 mg/kg d'aliment) | | | | | | | | |
| Lot 5 | 180 ± 52 | 840 ± 45 | 2,45 | 3,3 | 98000 ± 4200 | 136000 ± 6500 | diarrhéique | diarrhéique |
| Traitement au thymol seul (75 mg/kg d'aliment) | | | | | | | | |
| Lot 6 | 230 ± 33 | 1120 ± 45 | 1,65 | 1,72 | 7240 ± 650 | 5400 ± 340 | normal | normal |
| Salynomicine (SAL-P) (40 mg/kg) + carvacrol (75 mg/kg) | | | | | | | | |
| Lot 7 | 220 ± 41 | 1085 ± 56 | 1,70 | 1,78 | 9300 ± 570 | 6200 ± 450 | normal | normal |
| Salynomicine (SAL-P) (40 mg/kg) + thymol (75 mg/kg) | | | | | | | | |

Il apparaît clairement du tableau 1 que les compositions selon l'invention ont une action antiparasitaire remarquable sur les deux souches d'Eimeria étudiées en comparaison avec la salynomicine seule et avec le carvacrol seul ou le thymol seul.

En effet, la potentialisation de la salynomicine par le carvacrol et le thymol permet d'obtenir in vivo des résultats surprenants quant à l'augmentation importante de l'activité antiparasitaire de la salynomicine.

En effet, tous les animaux infectés traités soit avec la salynomicine seule, soit le carvacrol seul, soit le thymol seul restent diarrhéiques quinze jours après la fin du traitement et leur gain de poids est très faible par rapport au lot de poulets témoins non infectés et non traités.

En revanche, lorsque les animaux ont été traités avec les compositions de l'invention, leur gain de poids, indice de consommation et aspect des fientes est équivalent à ces mêmes critères chez les témoins non infectés et non traités.

### EXEMPLE 2 : Action antipaludéenne propre de l'eugénol et du carvacrol et potentialisation de l'artésunate.

Test in vitro : Détermination de l'IC50 (concentration capable de provoquer une inhibition de croissance de 50% du parasite)

L'expérience a été menée avec quatre clones de *Plasmodium falciparum* agent du paludisme; maladie parasitaire appelée aussi Malaria. Ces clones sont appelés 3d7, HB3, Dd2 et 7G8. Ces clones représentent des modèles de référence de laboratoire couramment utilisés pour tester l'activité antipaludéenne des drogues.

Le *Plasmodium falciparum* est cultivé sur des globules rouges humains. Le test est réalisé sur des plaques de 96 puits dans lesquels les globules rouges parasités (synchronisés au sorbitol) sont mis en contact avec les différents traitements pendant 72 heures à 37°C dans une étuve à atmosphère contrôlée (5% CO₂, 1% 0₂ et 94%N₂). La croissance de l'agent pathogène est mesurée par la détermination de la quantité de son ADN fluorescent en présence de SybrGreen.

L'antiparasitaire testé est l'artésunate, qui fait partie des agents antiparasitaires les plus efficaces. Une composition pharmaceutique antiparasitaire selon l'invention a été fabriquée en mélangeant l'artésunate à différentes concentrations, avec le carvacrol ou l'eugénol à des concentrations infra-inhibitrices respectives de 0,05mM et 0,2 mM. Ces concentrations sont deux à six fois (selon le clone) plus faibles que la CI50 du carvacrol seul et de l'eugénol seul. Cette composition pharmaceutique est notée Artésunate P eugénol, ou artésunate P carvacrol. La lettre P signifie potentialisé par l'eugénol ou par le carvacrol.

Dans chaque cas, l'activité antiparasitaire a été testée soit avec l'artésunate seul, soit avec le carvacrol seul, soit avec l'eugénol seul, soit avec la composition selon l'invention.

La CI 50 est déterminée par le logiciel HN NonLin V1.051Beta software de Harald Noedl en se basant sur les valeurs obtenus par fluorescence.

Le tableau 2 et le tableau 3 ci-après donnent les résultats de tests qui mesurent l'IC50 des différentes compositions :

**Tableau 2**

| Clones de *Plasmodium falciparum* en croissance | CI 50 Artésunate seul nM | CI 50 Composition selon l'invention Artésunate P carvacrol (nM artésunate) | CI 50 Carvacrol seul mM |
|---|---|---|---|
| Clone 3d7 | 2,14 | 1,025 | 0,33 |
| Clone HB3 | 2,28 | 1,15 | 0,22 |
| Clone Dd2 | 2,66 | 1,25 | 0,16 |
| Clone 7G8 | 1,00 | 0,47 | 0,11 |

**Tableau 3**

| Clones de *Plasmodium falciparum* en croissance | CI 50 Artésunate seul nM | CI 50 Composition selon l'invention Artésunate P eugénol (nM artésunate) | CI 50 Eugénol seul mM |
|---|---|---|---|
| Clone 3 d7 | 2,14 | 1,025 | 1,25 |
| Clone HB3 | 2,28 | 1,15 | 0,66 |
| Clone Dd2 | 2,66 | 1,40 | 0,66 |
| Clone 7G8 | 1,25 | 0,50 | 0,40 |

Il apparaît clairement des tableaux 2 et 3 que le carvacrol et l'eugénol possèdent une activité antipaludéenne remarquable propre à eux à des concentrations assez faibles.

Il apparaît aussi du tableau 2 et du tableau 3 que la composition selon l'invention a une action antipaludéenne remarquable sur ces clones de sensibilité variables en comparaison avec l'artésunate seul ou le carvacrol ou l'eugénol seuls aux concentrations infra-inhibitrices utilisées dans ce test.

En effet, on constate qu'en utilisant une concentration de 0,05 mM de carvacrol et 0,2 mM d'eugénol soit des concentrations environ 2 à 6 fois inférieures à la CI 50 du carvacrol seul ou de l'eugénol seul, la concentration en artésunate permettant d'obtenir 50% d'inhibition est pratiquement réduite de moitié.

On notera que l'action antipaludéenne de l'artésunate potentialisé selon l'invention n'est que deux fois meilleure que celle de l'artésunate seul dans cet exemple car les quatre clones sont déjà sensibles à l'artésunate seul. Malgré cela, la potentialisation a permis de doubler l'activité antipaludéenne.

### Exemple 3 : Action antipaludéenne propre de l'eugénol et potentialisation de la chloroquine.

Test in vitro : Détermination de l'IC50 (concentration capable de provoquer une inhibition de croissance de 50% du parasite)

L'expérience a été menée avec les mêmes clones de *Plasmodium falciparum* que l'exemple 2 (3d7, HB3, Dd2 et 7G8). Les deux premiers clones (3d7 et HB3) étant sensibles et les deux autres (Dd2 et 7G8) étant résistants à la chloroquine.

L'antiparasitaire testé est la chloroquine, qui fait parti des agents antiparasitaires les plus utilisés. Une composition pharmaceutique antiparasitaire selon l'invention a été fabriquée en mélangeant la chloroquine à différentes concentrations, avec l'eugénol a une concentration infra-inhibitrice de 0,2 mM deux à six fois (selon les clones) inférieure à la CI 50 de l'eugénol seul. Cette composition pharmaceutique est notée chloroquine P eugénol. La lettre P signifie potentialisé par l'eugénol.

Dans chaque cas, l'activité antiparasitaire a été testée soit avec la chloroquine seule, soit avec l'eugénol seul, soit avec la composition selon l'invention.

La méthode de détermination des CI50 utilisée est la même que pour l'exemple 2.

Le tableau 4 ci-après donne les résultats de tests qui mesurent l'IC50 des différentes compositions :

**Tableau 4**

| CI50 *Plasmodium falciparum* en croissance | CI50 Chloroquine seule nM | CI50 Composition selon l'invention Chloroquine P eugénol (nM chloroquine) | CI50 Eugénol seul mM |
|---|---|---|---|
| Clone 3 d7 | 22,31 | 13,2 | 1,25 |
| Clone HB3 | 37,07 | 15,3 | 0,66 |
| Clone Dd2 | 493,84 | 12,5 | 0,66 |
| Clone 7G8 | 445,17 | 12,5 | 0,40 |

Il apparaît clairement du tableau 4 que l'eugénol possède une activité antipaludéenne remarquable qui lui est propre à une concentration assez faible.

Il apparaît aussi du tableau 4 que la composition selon l'invention a une action antipaludéenne remarquable sur ces clones de sensibilité variables en comparaison avec la chloroquine seule ou l'eugénol seul.

En effet, on constate qu'en utilisant une concentration de 0,2 mM d'eugénol soit une concentration environ 2 à 6 fois inférieure à la CI 50 de l'eugénol seul, la concentration en chloroquine permettant d'obtenir 50% d'inhibition est pratiquement réduite de moitié pour les deux clones sensibles (3d7 et HB3) et de presque 20 fois pour les deux clones résistants (Dd2 et 7G8).

### Exemple 4 : Action antipaludéenne propre de l'eugénol et potentialisation de la méfloquine.

Test in vitro : Détermination de l'IC50 (concentration capable de provoquer une inhibition de croissance de 50% du parasite)

L'expérience a été menée avec les mêmes clones de *Plasmodium falciparum* qu'aux exemples 2 et 3 (3d7, HB3, Dd2 et 7G8). Les deux premiers clones (3d7 et HB3) étant sensibles et les deux autres (Dd2 et 7G8) étant résistants à la méfloquine.

L'antiparasitaire testé est la méfloquine, qui fait parti des agents antiparasitaires les plus utilisés. Une composition pharmaceutique antiparasitaire selon l'invention a été fabriquée en mélangeant la méfloquine à différentes concentrations, avec l'eugénol à une concentration infra-inhibitrice de 0,2 mM deux à six fois (selon les clones) inférieure à la CI 50 de l'eugénol seul. Cette composition pharmaceutique est notée méfloquine P eugénol. La lettre P signifie "potentialisé par l'eugénol".

Dans chaque cas, l'activité antiparasitaire a été testée soit avec la méfloquine seule, soit avec l'eugénol seul, soit avec la composition selon l'invention.

La méthode de détermination des CI50 utilisée est la même que pour les exemples 2 et 3.

Le tableau 5 ci-après donne les résultats de tests qui mesurent l'IC50 des différentes compositions :

**Tableau 5**

| CI50 *Plasmodium falciparum* en croissance | CI50 Méfloquine seule nM | CI50 Composition selon l'invention Méfloquine P eugénol nM méfloquine | CI50 Eugénol seul mM |
|---|---|---|---|
| Clone 3 d7 | 8,83 | 2,79 | 1,25 |
| Clone HB3 | 8,96 | 3,48 | 0,66 |
| Clone Dd2 | 33,75 | 3,6 | 0,66 |
| Clone 7G8 | 15,32 | 2,67 | 0,40 |

Il apparaît clairement du tableau 5 que l'eugénol possède une activité antipaludéenne remarquable propre à une concentration assez faible.

Il apparaît aussi du tableau 5 que la composition selon l'invention a une action antipaludéenne remarquable sur ces clones de sensibilité variables en comparaison avec la méfloquine seule ou l'eugénol seul.

En effet, on constate qu'en utilisant une concentration de 0,2 mM d'eugénol soit une concentration environ 2 à 6 fois inférieure à la CI 50 de l'eugénol seul, la concentration en méfloquine permettant d'obtenir 50% d'inhibition est pratiquement réduite 3 fois pour les deux clones sensibles (3d7 et HB3) et de 5 à 10 fois pour les deux clones résistants (Dd2 et 7G8).

### Exemple 5: Action antipaludéenne propre du carvacrol et potentialisation de l'artésunate

### Test in vivo :

Dans ce test, l'activité antipaludéenne propre du carvacrol de même que celle de l'artésunate potentialisé par le carvacrol ont été étudiées *in vivo* sur un modèle animal de malaria cérébral (neuropaludisme). Ce modèle de référence pour ce genre d'infection est réalisé sur des souris (CBA/J) infectées par Plasmodium bergheï ANKA, agent du neuropaludisme.

L'infection et le traitement sont effectués par voie intrapéritonéale.

Dans ce modèle, les animaux commencent à présenter les premiers signes cliniques dés le quatrième ou cinquième jour (J4 ou J5) après l'infection. Les animaux ayant montré ces signes cliniques meurent dans les deux jours qui suivent (J6, J7).

Le test a comporté 5 lots de 10 souris organisés comme suit :
Lot 1 : animaux témoins infectés non traités
Lot 2 : animaux infectés traités au carvacrol (60 mg/kg de poids) deux fois par jour espacés de 12 heures.
Lot 3 : animaux infectés traités au carvacrol (40 mg/kg de poids) deux fois par jour espacés de 12 heures.
Lot 4 : animaux infectés traités à l'artésunate (40 mg par Kg de poids) une fois par jour
Lot 5 : animaux infectés traités à l'artésunate (40 mg par Kg de poids) une fois par jour et au carvacrol (40 mg/kg de poids) deux fois par jour.

Les traitements ont commencé le cinquième jour après l'infection (J5) et se sont poursuivis pendant trois jours (J5, J6 et J7).

Les résultats sont présentés au tableau 6 suivant :

**Tableau 6**

| | % de mortalité | Durées de vie des animaux morts (jours) | % de survivants à J12 |
|---|---|---|---|
| Lot 1(témoin) | 100 | 5,5 | na |
| Lot 2(carvacrol 120) | 20 | 7,5 | 80 |
| Lot 3(Carvacrol 80) | 100 | 7,5 | na |
| Lot 4 (Artésunate 40) | 100 | 7 | na |
| Lot 5 (Artésunate 40 et Carvacrol 80) | 0 | Na* | 100 |

| | | | |
|---|---|---|---|
| * na = non applicable | | | |

Il apparaît clairement du tableau 6 que le carvacrol seul, administré deux fois par jour à la dose de 60 mg par Kg de poids de l'animal est capable de protéger 80 % des animaux traités. Ce qui montre l'existence d'une activité antipaludéenne remarquable du carvacrol seul à la dose utilisée (120 mg par Kg de poids par 24h).

Un traitement au carvacrol deux fois par jour à 40 mg par kg ne donne pas de protection mais permet de prolonger légèrement la durée de vie des animaux traités, ce qui montre une action thérapeutique partiellement efficace de ce traitement à la dose utilisée (80 mg par kg de poids par 24h).

L'artésunate seul (40 mg par Kg de poids par 24h) ne protége pas non plus mais prolonge la durée de vie des animaux infectés, ce qui signifie une activité thérapeutique partiellement efficace.

L'association de l'artésunate (40 mg par Kg) une fois par jour avec le carvacrol (40 mg par Kg) deux fois par jour donne une protection de 100% des animaux au-delà de la durée pendant laquelle le neuropaludisme peut survenir soit au-delà de J12.

Il apparaît clairement de ces résultats que le carvacrol seul possède une activité antipaludéenne remarquable à la dose (60 mg par Kg deux fois par jour).

Il apparaît aussi clairement de ces résultats que la composition selon l'invention a une action antipaludéenne remarquable en comparaison avec l'artésunate seul ou le carvacrol seul.

## Revendications

1. Composition pharmaceutique **caractérisée en ce qu'**elle comprend :
- au moins une première substance thérapeutiquement active choisie parmi le thymol, l'eugénol et le carvacrol, et
- au moins une seconde substance thérapeutiquement active qui est un antiparasitaire choisi parmi la salinomycine, l'artésunate, la chloroquine et la méfloquine.

2. Composition selon la revendication 1, **caractérisée en ce que** l'antiparasitaire est la salinomycine.

3. Composition selon la revendication 1, **caractérisée en ce que** ladite première substance thérapeutiquement active est choisie parmi le carvacrol et l'eugénol, et l'antiparasitaire est choisi parmi l'artésunate, la méfloquine, et la chloroquine.

4. Composition selon l'une quelconque des revendications 1 à 3, **caractérisée en ce que** lesdites première et seconde substances thérapeutiquement actives sont mises en suspension dans une solution aqueuse d'agar.

5. Composition selon l'une quelconque des revendications 1 à 4, **caractérisée en ce qu'**elle ne contient pas de détergent ou de solvant.

6. Trousse **caractérisée en ce qu'**elle contient :
- au moins un premier récipient contenant une première substance thérapeutiquement active choisie parmi le thymol, l'eugénol et le carvacrol, et
- au moins un second récipient contenant une seconde substance thérapeutiquement active qui est un antiparasitaire choisi parmi la salinomycine, l'artésunate, la chloroquine et la méfloquine.

7. Trousse selon la revendication 6, **caractérisée en ce que** l'antiparasitaire est la salinomycine.

8. Trousse selon la revendication 6, **caractérisée en ce que** ladite première substance thérapeutiquement active est choisie parmi le carvacrol et l'eugénol, et l'antiparasitaire est choisi parmi l'artésunate, la méfloquine, et la chloroquine.

9. Composition selon l'une quelconque des revendications 1 à 5 ou trousse selon l'une quelconque des revendications 6 à 8 pour une utilisation dans le traitement d'une infection due à un parasite chez un patient.

10. Composition selon la revendication 3 ou trousse selon la revendication 8 pour une utilisation dans le traitement du paludisme chez un patient.

11. Composition ou trousse selon la revendication 9 ou 10, **caractérisée en ce que** les doses à administrer sont :
- entre 10 et 100 mg/kg de poids du patient/jour pour la première substance thérapeutiquement active, et
- entre 20 et 100 mg/kg de poids du patient/jour pour la seconde substance thérapeutiquement active de ladite composition.

## Claims

1. A pharmaceutical composition **characterized in that** it comprises:
- at least one first therapeutically active substance selected from the group consisting of thymol, eugenol and carvacrol, and
- at least one second therapeutically active substance which is an antiparasitic agent selected from the group consisting of salinomycin, artesunate, chloroquine and mefloquine.

2. Composition according to claim 1, **characterized in that** the antiparasitic agent is salinomycin.

3. Composition according to claim 1, **characterized in that** said first therapeutically active substance is selected from the group consisting of carvacrol and eugenol, and said antiparasitic agent is selected from the group consisting of artesunate, mefloquine and chloroquine.

4. Composition according to any one of claims 1 to 3, **characterized in that** said first and second therapeutically active substances are suspended in an aqueous agar solution.

5. Composition according to any one of claims 1 to 4, **characterized in that** said composition does not include any detergent or solvent.

6. Kit **characterized in that** it comprises:
- at least one first container containing a first therapeutically active substance selected from the group consisting of thymol, eugenol and carvacrol, and
- at least one second container containing a second therapeutically active substance which is an antiparasitic agent selected from the group consisting of salinomycin, artesunate, chloroquine and mefloquine.

7. Kit according to claim 6, **characterized in that** the antiparasitic agent is salinomycin.

8. Kit according to claim 6, **characterized in that** said first therapeutically active substance is selected from the group consisting of carvacrol and eugenol, and said antiparasitic agent is selected from the group consisting of artesunate, mefloquine and chloroquine.

9. Composition according to any one of claims 1 to 5 or kit according to any one of claims 6 to 8 for use for the treatment of an infection caused by a parasite in a patient.

10. Composition according to claim 3 or kit according to claim 8 for use for the treatment of malaria in a patient

11. Composition or kit according to claim 9 or 10, **characterized in that** doses to be administrated are:
- between 10 and 100 mg/kg of body weight/day of the first therapeutically active substance, and
- between 20 and 100 mg/kg of body weight/day of the second therapeutically active substance of said composition.

## Patentansprüche

1. Pharmazeutische Zusammensetzung, **dadurch gekennzeichnet, dass** sie umfasst:
- wenigstens eine erste therapeutisch aktive Substanz, ausgewählt aus Thymol, Eugenol und Carvacrol, und
- wenigstens eine zweite therapeutisch aktive Substanz, die ein Antiparasitikum ist, ausgewählt aus Salinomycin, Artesunat, Chloroquin und Mefloquin.

2. Zusammensetzung gemäß Anspruch 1, **dadurch gekennzeichnet, dass** das Antiparasitikum Salinomycin ist.

3. Zusammensetzung gemäß Anspruch 1, **dadurch gekennzeichnet, dass** besagte erste therapeutisch aktive Substanz aus Carvacrol und Eugenol ausgewählt ist und das Antiparasitikum aus Artesunat, Mefloquin und Chloroquin ausgewählt ist.

4. Zusammensetzung gemäß einem der Ansprüche 1 bis 3, **dadurch gekennzeichnet, dass** besagte erste und zweite therapeutisch aktive Substanz in einer wässrigen Agarlösung suspendiert sind.

5. Zusammensetzung gemäß einem der Ansprüche 1 bis 4, **dadurch gekennzeichnet, dass** sie kein Detergens oder Lösemittel enthält.

6. Kit, **dadurch gekennzeichnet, dass** er enthält:
- wenigstens einen ersten Behälter, enthaltend eine erste therapeutisch aktive Substanz, ausgewählt aus Thymol, Eugenol und Carvacrol, und
- wenigstens einen zweiten Behälter, enthaltend eine zweite therapeutisch aktive Substanz, die ein Antiparasitikum ist, ausgewählt aus Salinomycin, Artesunat, Chloroquin und Mefloquin.

7. Kit gemäß Anspruch 6, **dadurch gekennzeichnet, dass** das Antiparasitikum Salinomycin ist.

8. Kit gemäß Anspruch 6, **dadurch gekennzeichnet, dass** besagte erste therapeutisch aktive Substanz aus Carvacrol und Eugenol ausgewählt ist und das Antiparasitikum aus Artesunat, Mefloquin und Chloroquin ausgewählt ist.

9. Zusammensetzung gemäß einem der Ansprüche 1 bis 5 oder Kit gemäß einem der Ansprüche 6 bis 8 zur Verwendung bei der Behandlung einer Parasiteninfektion bei einem Patienten.

10. Zusammensetzung gemäß Anspruch 3 oder Kit gemäß Anspruch 8 zur Verwendung bei der Behandlung der Malaria bei einem Patienten.

11. Zusammensetzung oder Kit gemäß Anspruch 9 oder 10, **dadurch gekennzeichnet, dass** die zu verabreichenden Dosen:
- zwischen 10 und 100 mg/kg Körpergewicht des Patienten/Tag für die erste therapeutisch aktive Substanz, und
- zwischen 20 und 100 mg/kg Körpergewicht des Patienten/Tag für die zweite therapeutisch aktive Substanz besagter Zusammensetzung liegen.
